# EUROPÄISCHE PATENTANMELDUNG

(11) **EP 3 048 138 A1**
(43) Veröffentlichungstag der Anmeldung: **27.07.2016**
(21) Anmeldenummer: 15151917.0
(22) Anmeldetag: 21.01.2015
(51) Int. Cl.: C09B 23/01, C09B 23/10, C09B 23/14

(54) **Gelbe Methinfarbstoffe**

(71) Anmelder: LANXESS Deutschland GmbH, 50569 Köln (DE)
(72) Erfinder: Borst, Dr. Hans-Ulrich, 50189 Elsdorf (DE); Linke, Frank, 51069 Köln (DE); Michaelis, Dr. Stephan, 51519 Odenthal (DE)

(57) **Zusammenfassung**

Die vorliegende Erfindung betrifft neue Methinfarbstoffe, Verfahren zu deren Herstellung und deren Verwendung zum Färben von Kunststoffen, insbesondere Polyamiden, wobei gelbe Färbungen mit verbesserten Lichtechtheiten und verbesserten Thermostabilitäten erhalten werden.

## Beschreibung

Die vorliegende Erfindung betrifft neue Methinfarbstoffe, Verfahren zu deren Herstellung und deren Verwendung zum Färben von Kunststoffen.

Obwohl es auf dem Markt bereits eine Vielzahl gelber Farbstoffe zur Kunststoffeinfärbung gibt, besteht dennoch Bedarf an neuen Farbstoffen mit verbesserten Eigenschaften. Insbesondere besteht weiterhin ein Verbesserungsbedarf der bekannten Farbstoffe hinsichtlich der beiden Eigenschaften Farbstärke und Thermostabilität. Dies gilt insbesondere für die Anwendung der Farbstoffe zum Massefärbung von Polyamid.

Das Massefärben von synthetischen Polyamiden stellt an die verwendeten Farbmittel höhere Anforderungen als das Massefärben anderer Kunststoffe. Die Schmelzpunkte der synthetischen Polyamide liegen entscheidend höher und auch die chemische Reaktivität der geschmolzenen Polyamide, insbesondere des Polyamids 6,6, ist wesentlich grösser, so dass die Hitzestabilität der verwendeten Farbmittel außerordentlich gut sein muss. Es gibt wenige Pigmente, welche diesen hohen Anforderungen genügen, insbesondere wenn zusätzlich auch eine hohe Lichtbeständigkeit verlangt wird.

In der DE-A 3543512 A1 (Bayplast Gelb G) werden Azofarblacke beschrieben, die zur Einfärbung von Polyamid in gelben Farbtönen verwendet werden können. Ebenfalls bekannt ist die Verwendung von Pigment Yellow 192. In EP-A 0074515 werden Nickelazobarbitursäurekomplexe offenbart, die ebenfalls zur Erzielung gelber Färbungen von Polyamid eingesetzt werden können.

Die Eigenschaften dieser, aus dem Stand der Technik bekannten gelben Farbmittel sind jedoch nicht ausreichend für die mittlerweile bestehenden technischen Anforderungen und sind insbesondere hinsichtlich ihrer Echtheiten, wie Licht- und Hitzebeständigkeit noch verbesserungsbedürftig.

Gegenstand der vorliegenden Erfindung sind neue Methinfarbstoffe der Formel (I) 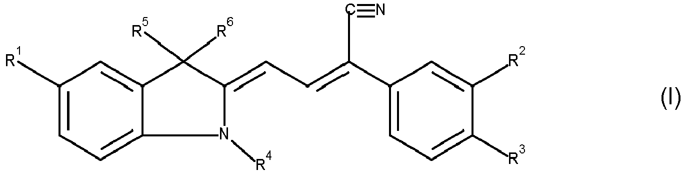 worin
R¹ für Wasserstoff, Halogen, COOH oder COOR⁷ steht,
R² für Wasserstoff, Halogen, CF₃, oder CN steht,
R³ für Wasserstoff, Halogen, COOR⁸ oder CN steht,
R⁴ für Alkyl oder Phenyl steht
und
R⁵ und R⁶ unabhängig voneinander für Alkyl stehen,
R⁷ für Alkyl steht
und
R⁸ für Alkyl steht.

Bevorzugt sind Farbstoffe der Formel (I),
worin
R¹ für Wasserstoff, Halogen, COOH oder COOR⁷ steht,
R² für Wasserstoff, Halogen, CF₃, oder CN steht,
R³ für Wasserstoff, Halogen, COOR⁸ oder CN steht,
R⁴ für geradkettiges oder verzweigtes C₁-C₄- Alkyl oder Phenyl steht
und
R⁵ und R⁶ unabhängig voneinander für geradkettiges oder verzweigtes C₁-C₄- Alkyl stehen,
R⁷ für geradkettiges oder verzweigtes C₁-C₄- Alkyl steht
und
R⁸ für geradkettiges oder verzweigtes C₁-C₄- Alkyl steht.

Besonders bevorzugt sind Farbstoffe der Formel (I), worin
R¹ für Wasserstoff, Fluor, Chlor, COOH oder COOCH₃ steht,
R² für Wasserstoff, Fluor, Chlor, CF₃, oder CN steht,
R³ für Wasserstoff, Chlor, COOCH₃ oder CN steht,
R⁴ für Methyl oder Phenyl steht
und
R⁵ und R⁶ für Methyl stehen.

Mit den erfindungsgemäßen Farbstoffen der Formel (I) lassen sich gelbe Einfärbungen von Kunststoffen, insbesondere von Polyamiden, erzielen, die sich überraschender Weise durch verbesserte Lichtechtheiten und verbesserte Thermostabilität gegenüber den bekannten, für diese Zwecke eingesetzten, gelben Farbstoffen auszeichnen.

Mit den erfindungsgemäßen Farbstoffen gelingt es, die bisher realisierten Eigenschaftsprofile bekannter gelber Farbstoffe für Kunststoffeinfärbungen deutlich zu übertreffen.

Weiterer Gegenstand der vorliegenden Erfindung ist die Verwendung der erfindungsgemäßen Farbstoffe der Formel (I) zum Massefärben von Kunststoffen. Dabei können die erfindungsgemäßen Farbstoffe einzeln oder in beliebiger Mischung untereinander eingesetzt werden.

Unter Massefärben werden hierbei insbesondere Verfahren verstanden, bei denen der Farbstoff in die geschmolzene Kunststoffmasse eingearbeitet wird, z.B. unter Zuhilfenahme eines Extruders, oder bei denen der Farbstoff bereits den Ausgangskomponenten zur Herstellung des Kunststoffes, z.B. Monomeren vor der Polymerisation, zugesetzt wird.

Besonders bevorzugte Kunststoffe sind Thermoplaste, beispielsweise Vinylpolymere, Polyester, Polyamide sowie Polyolefine, insbesondere Polyethylen und Polypropylen, Polycarbonate und Polyamid. Ganz besonders bevorzugt sind Polyamide, insbesondere Polyamid 6.6 und Polyamid 6.

Die Bezeichnung Polyamide wird im Sinne der vorliegenden Erfindung als Bezeichnung für synthetische, technisch verwendbare thermoplastische Kunststoffe verwendet und grenzt diese Stoffklasse damit von den chemisch verwandten Proteinen ab. Fast alle bedeutsamen Polyamide leiten sich von primären Aminen ab, denn die sich wiederholende Einheit besteht aus der funktionellen Gruppe -CO-NH-. Daneben existieren auch Polyamide von sekundären Aminen (-CO-NR-, R = organischer Rest). Als Monomere für die Herstellung der Polyamide dienen insbesondere Aminocarbonsäuren, Lactame und/oder Diamine und Dicarbonsäuren.

Polyamid 6.6 wird üblicher Weise aus Hexamethylendiamin (HMD) und Adipinsäure hergestellt. Es entsteht durch eine Polykondensation unter Wasserabspaltung.
Polyamid 6 ist erhältlich durch Ringöffnungspolymerisation von ε-Caprolactam mit Wasser als Starter.

Geeignete Vinylpolymere sind Polystyrol, Styrol-Acrylnitril-Copolymere, Styrol-Bu-tadien-Copolymere, Styrol-Butadien-Acrylnitril-Terpolymere, Polymethacrylat, Polyvinylchlorid u.a.

Geeignete Polyester sind beispielsweise Polyethylenterephthalate, Polycarbonate und Celluloseester.

Die zu färbenden Kunststoffe können einzeln oder in Gemischen untereinander, als plastische Massen oder Schmelzen vorliegen.

Bei ihrem Einsatz zum Massefärben von Kunststoffen werden die erfindungsgemäßen Farbstoffe (I) vorzugsweise in feinverteilter Form zur Anwendung gebracht, wobei Dispergiermittel mitverwendet werden können aber nicht müssen.

Bei ihrem Einsatz zum Massefärben von Kunststoffen können die erfindungsgemäßen Farbstoffe (I) beispielsweise direkt beim Prozess der Kunststoffherstellung nach der erfolgten Polymerisation eingesetzt werden. Dabei wird vorzugsweise mindestens ein erfindungsgemäßer Farbstoff (I) mit dem Kunststoffgranulat trocken vermischt oder vermahlen und dieses Gemisch beispielsweise auf Mischwalzen oder in Schnecken plastifiziert und homogenisiert. Man kann die erfindungsgemäßen Farbstoffe (I) aber auch der schmelzflüssigen Masse zugeben und durch Rühren homogen verteilen. Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Da die Farbstoffe (I) gegenüber Polymerisationskatalysatoren, insbesondere Peroxiden, beständig sind, ist es auch möglich, die erfindungsgemäßen Farbstoffe (I) den monomeren Ausgangsmaterialien für die Kunststoffherstellung, z. B. von Polymethylmethacrylat (PMMA) zuzusetzen und dann in Gegenwart von Polymerisationskatalysatoren zu polymerisieren. Dazu wird der Farbstoff vorzugsweise in den monomeren Komponenten gelöst oder mit ihnen innig vermischt.

Die erfindungsgemäßen Farbstoffe der Formel (I) werden vorzugsweise zum Färben der genannten Kunststoffe, insbesondere Polyamid, in Mengen von 0,0001 bis 1 Gew.-%, insbesondere 0,01 bis 0,5 Gew.-%, bezogen auf die Menge an Polymer eingesetzt.

Durch Zusatz von in den Polymeren unlöslichen Pigmenten, wie z.B. Titandioxid, können entsprechende wertvolle gedeckte Färbungen erhalten werden.

Titandioxid kann in einer Menge von 0,01 bis 10 Gew.-%, vorzugsweise 0,1 bis 5 Gew.-%, bezogen auf die Polymermenge, verwendet werden.

Gegenstand der vorliegenden Erfindung ist weiterhin ein Verfahren zum Massefärben von Kunststoffen, wobei mindestens ein Farbstoff der Formel (I) mit mindestens einem Kunststoff, vorzugsweise in Granulatform, trocken vermischt oder vermahlen wird und dieses Gemisch z.B. auf Mischwalzen oder in Schnecken plastifiziert und homogenisiert wird.

Man kann die erfindungsgemäßen Farbstoffe (I) aber auch der schmelzflüssigen Masse zugeben und durch Rühren homogen verteilen. Ebenfalls ist es möglich, die erfindungsgemäßen Farbstoffe (I) bei der Kunststoffherstellung den monomeren Ausgangskomponenten zuzusetzen und anschließend zu polymerisieren.

Das derart vorgefärbte Material kann dann wie üblich z.B. durch Verspinnen zu Borsten, Fäden usw. oder durch Extrusion oder im Spritzguss-Verfahren zu Formteilen weiterverarbeitet werden.

Nach dem erfindungsgemäßen Verfahren erhält man transparente bzw. gedeckte brillante gelbe Färbungen mit sehr guter Hitze- und Lichtbeständigkeit.

Zur Durchführung des erfindungsgemäßen Verfahrens können auch Mischungen der erfindungsgemäßen Farbstoffe der Formel (I) mit anderen Farbstoffen und/oder anorganischen und/oder organischen Pigmenten eingesetzt werden.

Weiterer Gegenstand der vorliegenden Erfindung ist ein Verfahren zur Herstellung der erfindungsgemäßen Farbstoffe der Formel (I).

Die erfindungsgemäßen Farbstoffe der Formel (I) können in an sich bekannter Weise hergestellt werden, indem man mindestens einen Aldehyd der Formel (II) 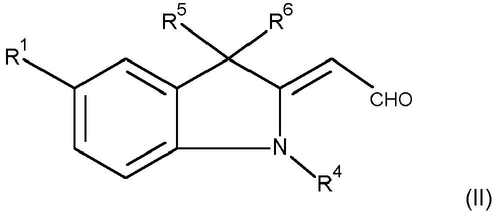 worin
R¹, R⁴, R⁵ und R⁶ die für Formel (I) angegebenen allgemeinen und bevorzugten Bedeutungen haben,
mit mindestens einem Phenylacetonitril -Derivat der Formel (III) 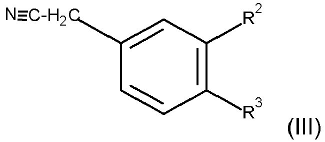 worin
R² und R³ die für Formel (I) angegebenen allgemeinen und bevorzugten Bedeutungen haben,
umsetzt.

Das Verfahren zur Herstellung der erfindungsgemäßen Farbstoffe (I) durch Umsetzung der Aldehyde der Formel (II) mit den Phenylacetonitril-Derivaten der Formel (III) kann in an sich bekannter Art und Weise durchgeführt werden.

Das Verfahren zur Herstellung der erfindungsgemäßen Farbstoffe (I) wird im Allgemeinen bei einer Temperatur im Bereich von -10 bis 180°C, bevorzugt von 0 bis 100°C und besonders bevorzugt von 10 bis 90°C durchgeführt.

Das Verfahren zur Herstellung der erfindungsgemäßen Farbstoffe (I) wird im Allgemeinen bei einem Druck von 0,9 bis 1,1 Bar, vorzugsweise bei Normaldruck durchgeführt.

Das Verfahren zur Herstellung der erfindungsgemäßen Farbstoffe (I) kann in Gegenwart von mindestens einem Lösungsmittel durchgeführt werden. Geeignete Lösungsmitte sind beispielsweise solche aus der Reihe der Alkohole und Formamide. Vorzugsweise wird das Verfahren zur Herstellung der erfindungsgemäßen Farbstoffe (I) in Gegenwart von mindestens einem Alkohol aus der Reihe Methanol, Ethanol, Propanol, und/oder mindestens einem Formamid aus der Reihe Diemethylformamid und Diethylformamid, besonders bevorzugt in Gegenwart von Methanol und/oder Dimethylformamid durchgeführt.

Das Verfahren zur Herstellung der erfindungsgemäßen Farbstoffe (I) kann in Gegenwart von mindestens einer Base durchgeführt werden. Geeignete Basen sind beispielsweise Alkalihydroxide und Alkalialkoholate. Bevorzugt ist die Verwendung von Lithiumhydroxid, Natriumhydroxid, Kaliumhydroxid und/oder Kalium-tert.-butylat, besonders bevorzugt von Natriumhydroxid und/oder Kalium-tert.-butylat.

Im Allgemeinen wird das Verfahren zur Herstellung der erfindungsgemäßen Farbstoffe (I) so durchgeführt, dass zunächst der Aldehyd (II) vorlegt und das Phenylacetonitril-Derivat (III) hinzugefügt wird und nach erfolgter Umsetzung die Verbindungen der Formel (I) isoliert wird. Die Isolierung kann durch übliche Verfahren, vorzugsweise durch Filtration, erfolgen. Das erhaltene Reaktionsprodukt kann gegebenenfalls durch weitere Verfahrensschritte wie Waschen und Trocknen aufgearbeitet werden.

Zur Durchführung des Verfahrens wird im Allgemeinen pro Mol an Aldehyd (II) 0,8 bis 1,5 Mol an Phenylacetonitril-Derivat (III) eingesetzt. Bevorzugt wird pro Mol an Aldehyd (II) 0,9 bis 1,1 Mol an Phenylacetonitril-Derivat (III) eingesetzt und besonders bevorzugt wird pro Mol an Aldehyd (II) 1 Mol an Phenylacetonitril-Derivat (III) eingesetzt.

Phenylacetonitril-Derivate der Formel (III) sind bekannt und können beispielsweise als Handelsprodukte der Firma Alfa Acer bezogen werden.

Die Aldehyde der Formel (II) sind neu und ebenfalls Gegenstand der vorliegenden Erfindung.

Sie können beispielsweise in dem Fachmann bekannter Art und Weise, in einer zweistufigen Synthese hergestellt werden. Dabei wird in einer ersten Stufe a) mindestens ein Indol-Derivat der Formel (IV) 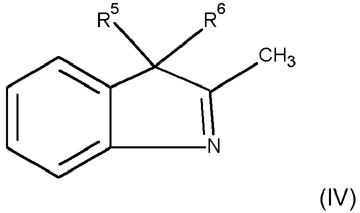 worin
R⁵ und R⁶ die für Formel (I) angegebenen allgemeinen und bevorzugten Bedeutungen haben,
mit mindestens einem Alkylierungsreagenz umgesetzt, und anschließend in einer zweiten Stufe b) das Zwischenprodukt der ersten Stufe mit mindestens einem Formylierungsreagenz umgesetzt.

Umsetzungen der in Stufe b) beschrieben Art sind ist in der Literatur unter dem Namen Vilsmeier-Reaktion bekannt.

Im Allgemeinen wird die Umsetzung in Stufe a) so durchgeführt, dass man das Indol-Derivat der allgemeinen Formel (IV) vorlegt und gegebenenfalls in Gegenwart eines Lösungsmittels das Alkylierungsmittel hinzufügt.

Die erste Stufe a) der Umsetzung wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe a) wird im Allgemeinen bei einem Druck von 0,9 bis 1,1 Bar, vorzugsweise bei Normaldruck durchgeführt.

Die Umsetzung in Stufe a) kann in Gegenwart von mindestens einem Lösungsmittel durchgeführt werden. Geeignete Lösungsmitte sind beispielsweise solche aus der Reihe der Alkohole und Wasser. Vorzugsweise wird die Umsetzung in Stufe a) in Gegenwart von Wasser als Lösungsmittel durchgeführt.

Als Alkylierungsreagenz geeignet sind prinzipiell alle bekannten Alkylierungsreagenzien (siehe z. B. K. Schwetlick, Organikum, VEB Deutscher Verlag der Wissenschaften, Berlin, 15. Auflage 1977, Seite 260, 253, 674), wie beispielsweise Dimethylsulfat, Methyliodid oder Diazomethan. Bevorzugt ist die Verwendung von Dimethylsulfat.

Im allgemeinen wird pro Mol an Indol-Derivat mindestens ein Mol an Alkylierungsreagenz eingesetzt. Abhängig von der Struktur des Indol-Derivats kommen, entsprechend der vorgegebenen Stöchiometrie, auch höhere Molmengen zum Einsatz. Vorzugsweise wird pro Mol an Indol-Derivat (IV) 0,9 bis 1,1 Mol, besonders bevorzugt 1 Mol an Alkylierungsreagenz eingesetzt.

Das in Stufe a) hergestellte Zwischenprodukt kann durch übliche Verfahren, beispielsweise durch Filtration isoliert werden. Vorzugsweise wird das in Stufe a) hergestellte Zwischenprodukt ohne Isolierung direkt weiter in der anschließenden Stufe b) umgesetzt.

Im Allgemeinen wird die Umsetzung in Stufe b) so durchgeführt, dass man die alkylierte Verbindung aus der ersten Stufe a) in Form der erhaltenen Reaktionslösung vorlegt und das Formylierungsreagenz, gegebenenfalls in Gegenwart mindestens eines Lösungsmittels, hinzufügt und anschließend den so hergestellten Aldehyd der Formel (II), gegebenenfalls durch Zugabe einer geeigneten Menge eines geeigneten Fällungsmittels ausfällt, und anschließen den Aldehyd der Formel (II) durch übliche Verfahren, beispielsweise durch Filtration, isoliert.

Die Umsetzung in Stufe b) wird im Allgemeinen bei einer Temperatur im Bereich zwischen 10 und 80°C bevorzugt von 20 bis 70°C und besonders bevorzugt von 30 bis 60°C durchgeführt.

Die Umsetzung in Stufe b) wird im Allgemeinen bei einem Druck 0,9 bis 1,1 Bar, vorzugsweise bei Normaldruck durchgeführt.

Die Umsetzung in Stufe b) kann in Gegenwart von mindestens einem Lösungsmittel durchgeführt werden. Geeignete Lösungsmitte sind beispielsweise Formamide. Bevorzugt sind Dimethylformamid, und Diethylformamid, besonders bevorzugt ist die Verwendung von Dimethylformamid. Bei Verwendung von Dimethylformamid ist es insbesondere bevorzugt dieses im Überschusses einzusetzen, wobei das Dimethylformamid dann zugleich als Formylierungsreagenz und als Lösungsmittel dient.

Als Formylierungsreagenz wird in Stufe b) im Allgemeinen eine Mischung aus mindestens einem Formamid und mindestens einem Phosporsäurechlorid eingesetzt.
Bevorzugte Formamide sind Dimethylformamid, Diethylformamid und Dibutylformamid. Bevorzugtes Phosporsäurechlorid ist Phosphoroxychlorid.

Besonders bevorzugt wird als Formylierungsreagenz eine Mischung aus Dimethylformamid und Phosphoroxychlorid eingesetzt.

Im Allgemeinen wird pro Mol an alkylierter Verbindung aus Stufe 1 mindestens ein Mol an Formylierungsreagenz eingesetzt, bevorzugt 1,1 bis 1,5 Mol und besonders bevorzugt 1,1 bis 1 Mol.

Geeignete Fällungsmittel sind beispielsweise Alkohole wie Methanol und/oder Ethanol.

Bevorzugt wird als Fällungsmittel Methanol und/oder Ethanol, insbesondere Methanol eingesetzt.

Die Indol-Derivate der Formel (IV) sind dem Fachmann bekannt. Sie können in an sich bekannter Art und Weise in einer zweistufigen Synthese hergestellt werden durch Umsetzung eines Anilin-Derivats der Formel (V) 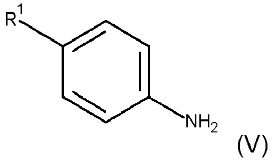 worin
R¹ die für Formel (I) angegebene allgemeine und bevorzugte Bedeutung hat,
mit einem Diazotierungsreagenz und anschließender Umsetzung unter Ringschluss mit einem Keton der Formel (VI) 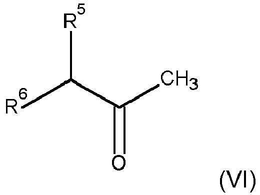 worin
R⁵ und R⁶ die für Formel (I) angegebene allgemeine und bevorzugte Bedeutung haben. Die Diazotierungsreaktion wird im allgemeinen durchgeführt, indem man das Anilin-Derivat vorlegt und das Diazotierungsreagenz bei einer Temperatur im Bereich zwischen 0 bis 10°C , bei Normaldruck in einem wässerigen Medium hinzufügt.

Als Diazotierungsreagenz kommt prinzipiell jedes geeignete Diazotierungsreagenz in Frage. Bevorzugt ist die Verwendung einer wässerigen Natriumnitrit-Lösung.

Im Allgemeinen wird das Diazotierungsreagenz in einer Menge von mindestens zwei Mol bezogen auf das Anilin-Derivat (V) eingesetzt.

Die Ringschluss-Reaktion mit dem Keton der Formel (VI) wird in an sich bekannter Art und Weise einer Eintopf-Reaktion durchgeführt, indem man das Diazoniumsalz des Anilin-Derivats (V) zum Hydrazon reduziert und das Hydrazon mit dem Keton der allgemeinen Formel (VI) umsetzt, bevorzugt bei einer Temperatur im Bereich von 40 bis 100°C, bevorzugt in wässriger Lösung, und anschließend das Indol-Derivat der Formel (IV) durch übliche Verfahren, vorzugsweise Filtration, isoliert und wäscht.

Die Anilin-Derivate der Formel (V) sowie die Ketone der Formel (VI) sind bekannt und können als Handelsprodukte bezogen werden, beispielsweise von den Firmen Alfa Acer oder Sigma-Aldrich.

Die Erfindung wird erläutert, jedoch nicht beschränkt durch die folgenden Beispiele, in denen die Teile sich auf das Gewicht beziehen und Prozentangaben Gewichtsprozente (Gew.-%) bedeuten.
1) Herstellung der erfindungsgemäßen Verbindungen der Formel (I)

### Beispiel 1

### Herstellung der erfindungsgemäßen Verbindung

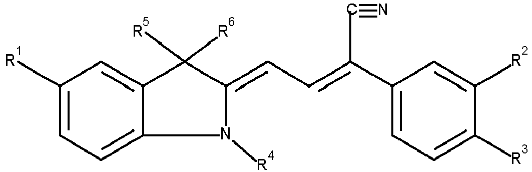

mit R¹ = -COOCH₃, R² und R³ = Cl, R⁴ = -CH₃ und R⁵ und R⁶ = -CH₃

In eine Vorlage von 100 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 18,6 g (= 0,1 Mol) 3,4-Dichlorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser mit einer Temperatur von 90°C gewaschen. Das gewaschene Produkt wurde im Vakuumtrockenschrank bei einer Temperatur von 80°C und einem Druck von 200 mbar getrocknet.
Ausbeute: 32,5 g (entspricht 76% der Theorie), Schmelzpunkt 241 °C

### Beispiel 2

### Herstellung der erfindungsgemäßen Verbindung

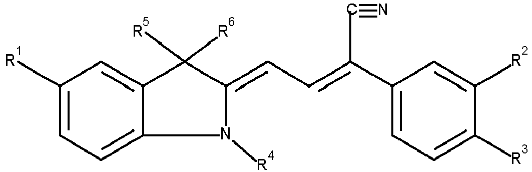

mit R¹ = -COOCH₃, R² = H, R³ = Cl, R⁴ = -CH₃ und R⁵ und R⁶ = -CH₃

In eine Vorlage von 100 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 15,2 g (= 0,1 Mol) 4-Chlorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 31,0 g (entspricht 79% der Theorie), Schmelzpunkt 199°C

### Beispiel 3

### Herstellung der erfindungsgemäßen Verbindung

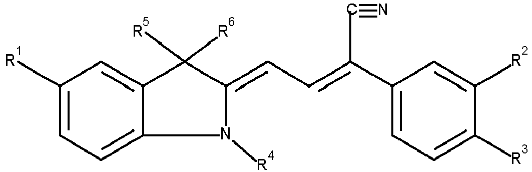

mit R¹ = -COOCH₃, R² = Cl, R³ = H, R⁴ = -CH₃ und R⁵ und R⁶ = -CH₃

In eine Vorlage von 100 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 15,2 g (= 0,1 Mol) 3-Chlorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6

Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 29,5 g (entspricht 75% der Theorie), Schmelzpunkt 130°C

### Beispiel 4

### Herstellung der erfindungsgemäßen Verbindung

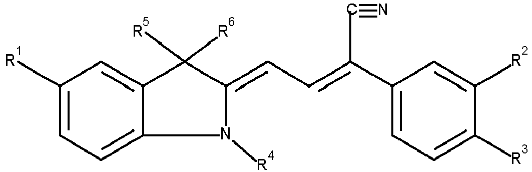

mit R¹ = -COOCH₃, R² = H, R³ = -COOCH₃, R⁴ = -CH₃ und R⁵ und R⁶ = -CH₃

In eine Vorlage von 200 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 17,5 g (= 0,1 Mol) Methyl 4-(cyanomethyl)benzoat eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 31,2 g (entspricht 75% der Theorie), Schmelzpunkt 246°C

### Beispiel 5

### Herstellung der erfindungsgemäßen Verbindung

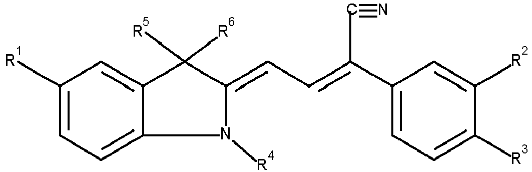

mit R¹ = -COOCH₃, R² = H, R³ = CN, R⁴ = -CH₃ und R⁵ und R⁶ = -CH₃

In eine Vorlage von 200 ml Methanol wurden 25,9 g (= 0,1 Mol) Aldehyd der Formel (II) mit R¹ = -COOCH₃, R⁴ = -CH₃ und R⁵ und R⁶ = -CH₃sowie 14,2 g (= 0,1 Mol) 4-Cyanophenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 26,8 g (entspricht 70% der Theorie), Schmelzpunkt 269°C

### Beispiel 6

### Herstellung der erfindungsgemäßen Verbindung

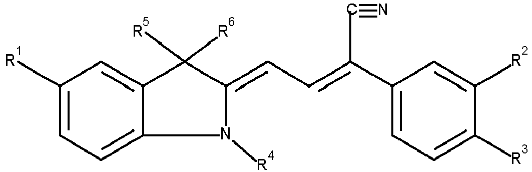

mit R¹ = -COOCH₃, R² = F, R³ = H, R⁴ = -CH₃ und R⁵ und R⁶ = -CH₃

In eine Vorlage von 200 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 13,5 g (= 0,1 Mol) 3-Fluorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 24,5 g (entspricht 65% der Theorie), Schmelzpunkt 311 °C

### Beispiel 7

### Herstellung der erfindungsgemäßen Verbindung

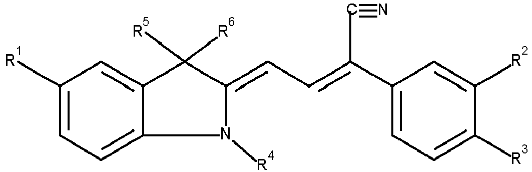

mit R¹ = -COOCH₃, R² = CF₃ , R³ = H, R⁴ = -CH₃ und R⁵ und R⁶ = -CH₃

In eine Vorlage von 200 ml Methanol wurden 25,9 g (= 0,1 Mol) Aldehyd der Formel (II) mit R¹ = -COOCH₃, R⁴ = -CH₃ und R⁵ und R⁶ = -CH₃ sowie 13,5 g (= 0,1 Mol) 3-(Trifluormethyl)phenylacetonitril eingetragen. Anschließend wurde mit ca. 1g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 24,5 g (entspricht 65% der Theorie), Schmelzpunkt 199°C

### Beispiel 8

### Herstellung der erfindungsgemäßen Verbindung

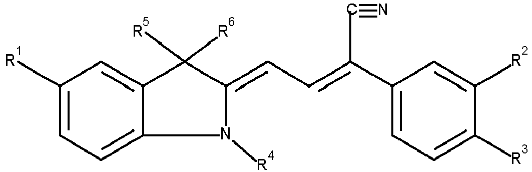

mit R¹ = -Cl, R² und R³ = Cl, R⁴ = -CH₃ und R⁵ und R⁶ = -CH₃

In eine Vorlage von 200 ml Methanol wurden 11,8 g (= 0,05 Mol) Aldehyd der Formel (II) mit R¹ = Cl, R⁴ = -CH₃ und R⁵ und R⁶ = -CH₃ - sowie 9,3 g (= 0,05 Mol) 3,4-Dichlorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 18,2 g (entspricht 90% der Theorie), Schmelzpunkt 239°C

### Beispiel 9

### Herstellung der erfindungsgemäßen Verbindung

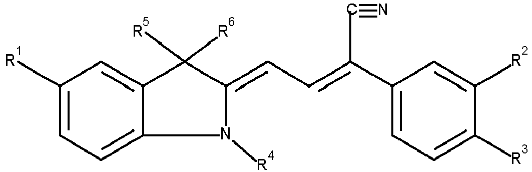

mit R¹ = -F , R² und R³ = Cl, R⁴ = -CH₃ und R⁵ und R⁶ = -CH₃

In eine Vorlage von 200 ml Methanol wurden 11,0 g (= 0,05 Mol) Aldehyd der Formel (II) mit R¹ = F, R⁴ = -CH₃ und R⁵ und R⁶ = -CH₃ sowie 9,3 g (= 0,05 Mol) 3,4-Dichlorphenylacetonitril eingetragen. Anschließend wurde mit ca. 1 g einer 50%-igen wässrigen Kaliumhydroxid-Lösung der pH-Wert auf etwa 10 eingestellt und der Reaktorinhalt auf eine Temperatur von 60°C erwärmt und ca. 6 Stunden nachgerührt. Danach wurde auf 25°C abgekühlt und das Reaktionsprodukt auf einer Nutsche isoliert. Der Filterkuchen wurde mit ca. 50 ml Methanol und ca. 500 ml Wasser (T = 90°C) gewaschen. Das gewaschene Produkt wurde im Vakuumtrockenschrank bei einer Temperatur von 80°C und einem Druck von 200 mbar getrocknet.
Ausbeute: 13,6 g (entspricht 70% der Theorie), Schmelzpunkt 238°C

### 2) Herstellung der Vorstufen

### Beispiel 10

### Herstellung eines Aldehyds der Formel (II)

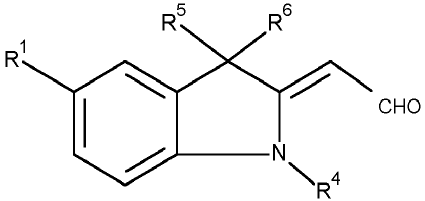

mit R¹ = -COOCH₃, R⁴ = -CH₃ und R⁵ und R⁶ = -CH₃
a) Herstellung der Diazotierung:
   In eine Vorlage von 270 g Salzsäure 30% wurden 139,9 g p-Aminobenzoesäure eingetragen und durch Kühlung von außen auf 0°C abgekühlt. Anschließend wurde mit ca. 174 g einer 40%-igen wässrigen Natriumnitrit Lösung versetzt. Nach ca. 30 minütigem Nachrühren wurde mit ca. 0,5 g Amidosulfonsäure der Nirit-Überschuss entfernt.
b) Herstellung des Hydrazons und Ringschluss:
   In eine Vorlage von 250 g Wasser und 660 g Natriumhydrogensulfit 39% wurde mit ca. 80 g einer 40%-igen wässrigen Natriumhydroxid-Lösung ein pH-Wert von ca. 6,5 eingestellt. Im Laufe von ca. 30 Min. wurde die oben beschriebene Diazotierungslösung überführt wobei mit ca. 100 g einer 40%-igen wässrigen Natriumhydroxid-Lösung ein pH-Wert von ca. 6,5 gehalten wurde. Bei einer Temperatur von 40°C wurde ca. 1 Stunde nachgerührt. Danach wurden 560 g Schwefelsäure (96%-ig) und anschließend 86,1 g Methylisopropylketon zugetropft. Der Reaktorinhalt wurde auf 70°C erwärmt und ca. 4 Stunden nachgerührt. Anschließend wurde der Reaktorinhalt auf 80°C erwärmt und ca. 4 Stunden nachgerührt. Danach wurde der Reaktorinhalt auf 25°C abgekühlt und mit ca. 800 g einer 40%-igen wässrigen Natriumhydroxid-Lösung auf einen pH-Wert von ca. 6,5 eingestellt. Nach 30 Minuten Nachrühren wurde das Reaktionsprodukt auf einer Nutsche isoliert und mit 2 Liter Wasser gewaschen.
c) Herstellung des Aldehyds:
   In eine Vorlage von 1200 g Wasser wurde der wasserfeuchte Presskuchen des Ringschlussproduktes aus Stufe b) eingetragen. Anschließend wurde mit ca. 70 g einer 40%-igen wässrigen Natriumhydroxid-Lösung ein pH-Wert von ca. 10 eingestellt. Im Laufe von ca. 1 Stunde wurden 325 g Dimethylsulfat zugetropft und dabei durch Zugabe von ca. 200 g einer 40%-igen wässrigen Natriumhydroxid-Lösung ein pH-Wert von ca. 8,5 gehalten. Der Reaktorinhalt wurde auf 40°C erwärmt und ca. 5 Stunden nachgerührt. Anschließend wurde der Reaktorinhalt auf 60°C erwärmt und ca. 1 Stunde nachgerührt. Die Reaktionsmischung wurde für ca. 1 Stunde stehen gelassen bis eine Phasentrennung erfolgt war. Dann wurde die wässrige Phase abgetrennt. Im Vakuum bei 80°C und 20 mbar wurde restliches Wasser aus der organischen Phase entfernt. Danach wurden zu der organischen Phase 310 g Dimethylformamid zugetropft. Anschließend wurden bei 40°C 263 g Phosphoroxychlorid im Laufe von 3 Std. zudosiert. Der Reaktorinhalt wurde für 5 Stunden nachgerührt. Anschließend wurde auf 20°C abgekühlt und mit 160 g Methanol versetzt. Anschließend wurde mit ca. 200 g einer 40%-igen wässrigen Natriumhydroxid-Lösung ein pH-Wert von ca. 11 eingestellt. Nach 60 Minuten Nachrühren wurde das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 mbar getrocknet.
   Ausbeute: 176,3 g (entspricht 68% der Theorie)

### Beispiel 11

### Herstellung eines Aldehyds der Formel (II)

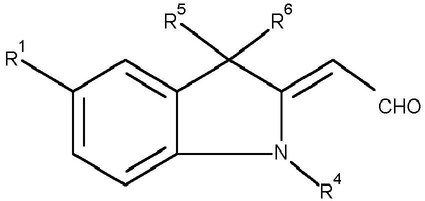

mit R¹ = Cl, R⁴ = CH₃ und R⁵ und R⁶ = CH₃
a) Herstellung der Diazotierung:
   In eine Vorlage von 268 g 30%-iger Salzsäure wurden 127,6 g 4-Chloranilin zugetropft und durch Kühlung von außen auf 0°C abgekühlt. Anschließend wurde mit ca. 174 g einer 40%-igen wässrigen Natriumnitrit Lösung versetzt. Nach ca. 30 Minuten Nachrühren wurde mit ca. 0,5 g Amidosulfonsäure der Nirit-Überschuss entfernt.
b) Herstellung des Hydrazons und Ringschluss:
   In eine Vorlage von 250 g Wasser und 660 g Natriumhydrogensulfit 39% wurde mit ca. 80 g einer 40%-igen wässrigen Natriumhydroxid-Lösung ein pH-Wert von ca. 6,5 eingestellt. Im Laufe von ca. 30 Minuten wurde die oben beschriebene Diazotierungslösung aus Stufe a) überführt wobei durch Zugabe von ca. 100 g einer 40%-igen wässrigen Natriumhydroxid-Lösung ein pH-Wert von ca. 6,5 gehalten wurde. Bei einer Temperatur von 40°C wurde ca. 1 Stunde nachgerührt. Danach wurden 560 g Schwefelsäure (96%-ig) und anschließend 86,1 g Methylisopropylketon zugetropft. Der Reaktorinhalt wurde auf 70°C erwärmt und ca. 4 Stunden nachgerührt. Anschließend wurde der Reaktorinhalt auf 80°C erwärmt und ca. 4 Stunden nachgerührt. Danach wurde der Reaktorinhalt auf 25°C abgekühlt und mit ca. 800 g einer 40%-igen wässrigen Natriumhydroxid-Lösung auf einen pH-Wert von ca. 6,5 eingestellt. Nach 30 Minuten Nachrühren wird das Reaktionsprodukt auf einer Nutsche isoliert und mit 2 Liter Wasser gewaschen.
c) Herstellung des Aldehyds:
   In eine Vorlage von 1200 g Wasser wurde der wasserfeuchte Presskuchen des Ringschlussproduktes aus Stufe b) eingetragen. Anschließend wurde mit ca. 5 g einer 40%-igen wässrigen Natriumhydroxid-Lösung ein pH-Wert von ca. 10 eingestellt. Im Laufe von ca. 1 Stunden wurden 153 g Dimethylsulfat zugetropft und dabei durch Zugabe von ca. 90 g einer 40%-igen wässrigen Natriumhydroxid-Lösung ein pH-Wert von ca. 8,5 gehalten. Der Reaktorinhalt wurde auf 40°C erwärmt und für ca. 5 Stunden nachgerührt. Anschließend wird der Reaktorinhalt auf 60°C erwärmt und für ca. 1 Stunde nachgerührt.
   Die Reaktionsmischung wurde für ca. 1 Stunde stehen gelassen bis eine Phasentrennung erfolgt war. Dann wurde die wässrige Phase abgetrennt. Im Vakuum bei 80°C und 20 mbar wurde restliches Wasser aus der organischen Phase entfernt. Danach wurden zu der organischen Phase 275 g Dimethylformamid zugetropft.
   Anschließend wurden bei 40°C 116 g Phosphoroxychlorid im Laufe von 3 Stunden zudosiert. Der Reaktorinhalt wurde 5 Stunden Nachgerührt, anschließend auf 20°C abgekühlt und mit 160 g Methanol versetzt. Anschließend wurde mit ca. 180 g einer 40%-igen wässrigen Natriumhydroxid-Lösung ein pH-Wert von ca. 11 eingestellt. Nach 60 Minuten Nachrühren wurde das Reaktionsprodukt auf einer Nutsche isoliert und mit 160 g Methanol und 2000 g Wasser gewaschen. Das gewaschene Produkt wurde im Vakuumtrockenschrank bei einer Temperatur von 80°C und einem Druck von 200 mbar getrocknet.
   Ausbeute: 141,4 g (entspricht 60% der Theorie)

### Beispiel 12

### Herstellung eines Aldehyds der Formel (II)

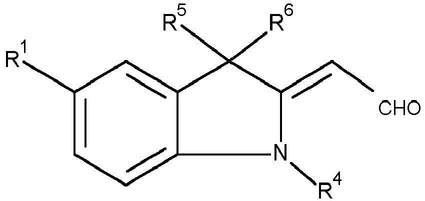

mit R¹ = F, R⁴ = -CH₃ und R⁵ und R⁶ = -CH₃
a) Herstellung der Diazotierung:
   In eine Vorlage von 375 g Salzsäure 30% wurden 155,5 g 4-Fluoranilin zugetropft und durch Kühlung von außen auf 0°C abgekühlt. Anschließend wurde mit ca. 244 g einer 40%-igen wässrigen Natriumnitrit-Lösung versetzt. Nach ca. 30 Minuten Nachrühren wurde mit ca. 0,5 g Amidosulfonsäure der Nirit-Überschuss entfernt.
b) Herstellung des Hydrazons und Ringschluss:
   In eine Vorlage von 250 g Wasser und 918 g Natriumhydrogensulfit-Lösung (39%-ig) wurde durch Zugabe von ca. 120 g einer 40%-igen wässrigen Natriumhydroxid-Lösung ein pH-Wert von ca. 6,5 eingestellt. Im Laufe von ca. 30 Mininuten wurde die oben beschriebene Diazotierungslösung aus Stufe a) überführt, wobei durch Zugabe von ca. 140 g einer 40%-igen wässrigen Natriumhydroxid-Lösung ein pH-Wert von ca. 6,5 gehalten wurde. Bei einer Temperatur von 40°C wurde ca. 1 Stunde nachgerührt. Danach wurden 776 g 96%-ige Schwefelsäure und anschließend 120,4 g Methylisopropylketon zugetropft. Der Reaktorinhalt wurde auf 70°C erwärmt und für ca. 4 Stunden nachgerührt. Anschließend wurde der Reaktorinhalt auf 80°C erwärmt und für ca. 4 Stunden nachgerührt. Danach wurde der Reaktorinhalt auf 25°C abgekühlt und mit ca. 1150 g einer 40%-igen wässrigen Natriumhydroxid-Lösung auf einen pH-Wert von ca. 6,5 eingestellt. Nach 30 Minuten Nachrühren wurde das Reaktionsprodukt auf einer Nutsche isoliert und mit 2 Liter Wasser gewaschen.
c) Herstellung des Aldehyds:
   In eine Vorlage von 1200 g Wasser wurde der wasserfeuchte Presskuchen des Ringschlussproduktes aus Stufe b) eingetragen. Anschließend wurde mit ca. 10 g einer 40%-igen wässrigen Natriumhydroxid-Lösung ein pH-Wert von ca. 10 eingestellt. Im Laufe von ca. 1 Stunde wurden 194 g Dimethylsulfat zugetropft und dabei durch Zugabe von ca. 120 g einer 40%-igen wässringen Natriumhydroxid-Lösung ein pH-Wert von ca. 8,5 gehalten. Der Reaktorinhalt wurde auf 40°C erwärmt und ca. 5 Stunden nachgerührt. Anschließend wurde der Reaktorinhalt auf 60°C erwärmt und ca. 1 Stunde nachgerührt.
   Die Reaktionsmischung wurde für ca. 1 Stunde stehen gelassen bis eine Phasentrennung erfolgt war. Dann wurde die wässrige Phase abgetrennt. Im Vakuum bei 80°C und 20 mbar wurde restliches Wasser aus der organischen Phase entfernt. Danach wurden zu der organischen Phase 350 g Dimethylformamid zugetropft. Anschließend wurden bei 40°C 147 g Phosphoroxychlorid im Laufe von 3 Stunden zudosiert. Der Reaktorinhalt wurde 5 Stunden Nachgerührt, anschließend auf 20°C abgekühlt und mit 160 g Methanol versetzt. Anschließend wurde durch Zugabe von ca. 200 g einer 40%-igen wässrigen Natriumhydroxid-Lösung ein pH-Wert von ca. 11 eingestellt. Nach 60 Minuten Nachrühren wurde das Reaktionsprodukt auf einer Nutsche isoliert und mit 160g Methanol und 2000g Wasser gewaschen. Das gewaschene Produkt wurde im Vakuumtrockenschrank bei einer Temperatur von 80°C und einem Druck von 200 mbar getrocknet.
   Ausbeute: 162,8 g (entspricht 53% der Theorie)

### Liste der zugekauften Einsatzstoffe:

| Bezeichnung: | Molgewicht | Cas. Nr. | Gehalt: | Hersteller: |
|---|---|---|---|---|
| p-Aminobenzoesäure | 137,2 | 150-13-0 | 98 | Sigma-Aldrich |
| | | | | |
| Methylisopropylketon | 86,1 | 563-80-4 | 99 | Sigma-Aldrich |
| Isopropyl methyl ketone | | | | |
| 4- Chloranilin | 127,6 | 106-47-8 | 98 | Sigma-Aldrich |
| 4- Fluoranilin | 111,1 | 371-40-4 | 99 | Alfa Acer |
| 3,4-Dichlorphenylacetonitril | 186,0 | 3218-49-3 | 98 | Alfa Acer |
| 4-Chlorphenylacetonitril | 151,6 | 140-53-4 | 98 | Alfa Acer |
| 3-Chlorphenylacetonitril | 151,6 | 1529-41-5 | 99 | Alfa Acer |
| Methyl 4-(cyanomethyl)benzoate | 175,2 | 76469-88-0 | 96 | Sigma-Aldrich |
| 4-Cyanophenylacetonitril | 142,2 | 876-31-3 | 97 | Alfa Acer |
| 3-Fluorphenylacetonitril | 135,1 | 501-00-8 | 98 | Alfa Acer |
| | | 10036-43-8 | | |
| 3-(Trifluormethyl)phenylacetonitril | 185,2 | 2338-76-3 | 97 | Sigma-Aldrich |

Die Ergebnisse der UV/VIS Messungen und Extinktionswerte für die erfindungsgemäßen Verbindungen der Beispiele 1 bis 9 sind in Tabelle 1 aufgeführt.

**Tabelle 1**

| Verbindung aus | Absorptionsmaximum UV/VIS-Spektrum¹⁾ | E 1/1-Wert²⁾ |
|---|---|---|
| Beispiel 1 | 427 nm | 2498 |
| Beispiel 2 | 419 nm | 1926 |
| Beispiel 3 | 425 nm | 1402 |
| Beispiel 4 | 440 nm | 1326 |
| Beispiel 5 | 445 nm | 1464 |
| Beispiel 6 | 419 nm | 1141 |
| Beispiel 7 | 422 nm | 1128 |
| Beispiel 8 | 430 nm | 1230 |
| Beispiel 9 | 428 nm | 1156 |

| | | |
|---|---|---|
| ¹⁾ Die UV/VIS-Absorptionsspektren der erfindungsgemäßen Verbindungen wurden alle im Lösungsmittel 1-Methoxy-2-propylacetat (CAS-Nr. 108-65-6) gemessen. ²⁾ Der angegebene E1/1-Wert ist ein fiktiver Extinktionswert, den man erhalten würde, wenn man eine 1 gewichtsprozentige Lösung der jeweiligen Verbindung (gelöst in 1-Methoxy-2-propylacetat) in einer Küvette der Schichtdicke von 1 cm vermessen würde. | | |

### 3) Anwendungstechnische Ergebnisse:

### A) Beschreibung der Prüfmethode "Thermostabilität"

In einem Taumelmischer wurden jeweils 2 g des zu prüfenden Farbstoffs mit 1998 g eines PA6 Granulates vom Typ Durethan B30S (Handelsprodukt der Lanxess Deutschland GmbH) mit 1% TiO2, welches 4 Stunden bei 80°C getrocknet wurde, gemischt. Dieses Gemisch wurde bei einer Massetemperatur von maximal 240°C auf einem Einschneckenextruder (Fa. Stork, 25mm Schnecke) extrudiert, mit Wasser gekühlt, mit einem Granulator der Fa. Sheer granuliert und 8 Stunden bei 80°C getrocknet. Die Hitzestabilität des erhaltenen Kunststoffgranulates wurde nach DIN EN 12877-2 ("Bestimmung der Beständigkeit der Farbe gegen Hitze beim Verarbeiten von Farbmitteln in Kunststoffen") (Methode A) auf einer Spritzgussmaschine geprüft. Als Standard wurde eine Probe bei 240°C mit einer Verweilzeit in der Schnecke von 2,5 Minuten hergestellt. Gegenüber dieser Standardprobe wurden die zu bestimmenden Proben koloristisch bewertet, die bei einer Verweilzeit von 5 Minuten und Temperaturen von 240-320°C hergestellt wurden. Proben mit einer Gesamtfarbabweichung von DE ≤ 3,0 wurden als stabil bei der angewandten Temperatur gewertet.

Die Ergebnisse der Bestimmung der Thermostabilität der erfindungsgemäßen Verbindungen der Beispiele 1 bis 9 sowie die der nicht erfindungsgemäßen Verbindungen des Standes der Technik sind in Tabelle 2 aufgeführt.

**Tabelle 2**

| Erfindungsgemäße Verbindung | Hitzestabil bis (°C) |
|---|---|
| Beispiel 1 | 300 |
| Beispiel 2 | 320 |
| Beispiel 3 | 320 |
| Beispiel 4 | 280 |
| Beispiel 5 | 260 |
| Beispiel 6 | 300 |
| Beispiel 7 | 280 |
| Beispiel 8 | 320 |
| Beispiel 9 | 320 |

### Nicht erfindungsgemäße Vergleichsverbindungen

| | |
|---|---|
| D.Y 201 (Macrolex Yellow 6G) | Entfärbung bei 240°C |
| S. Y. 93 (Macrolex Yellow 3G) | Entfärbung bei 240°C |
| S.Y 114 (Macrolex Yellow G) | 240°C |
| S.Y 160:1 (Macrolex Fluor. Yellow 10GN) | <240°C (DE 3,6 bei 240°C) |

## Patentansprüche

1. Methinfarbstoff der Formel (I) 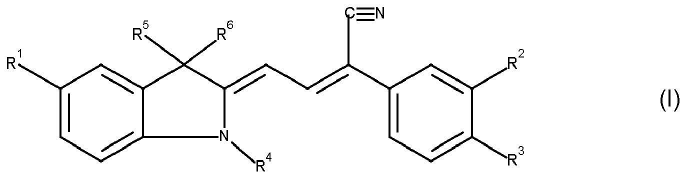 worin
R¹ für Wasserstoff, Halogen, COOH oder COOR⁷ steht,
R² für Wasserstoff, Halogen, CF₃, oder CN steht,
R³ für Wasserstoff, Halogen, COOR⁸ oder CN steht,
R⁴ für Alkyl oder Phenyl steht
und
R⁵ und R⁶ unabhängig voneinander für Alkyl stehen,
R⁷ für Alkyl steht
und
R⁸ für Alkyl
steht.

2. Methinfarbstoff gemäß Anspruch 1, **dadurch gekennzeichnet, dass**
R¹ für Wasserstoff, Halogen, COOH oder COOR⁷ steht,
R² für Wasserstoff, Halogen, CF₃, oder CN steht,
R³ für Wasserstoff, Halogen, COOR⁸ oder CN steht,
R⁴ für geradkettiges oder verzweigtes C₁-C₄- Alkyl oder Phenyl steht
und
R⁵ und R⁶ unabhängig voneinander für geradkettiges oder verzweigtes C₁-C₄- Alkyl stehen,
R⁷ für geradkettiges oder verzweigtes C₁-C₄- Alkyl steht
und
R⁸ für geradkettiges oder verzweigtes C₁-C₄- Alkyl
steht.

3. Methinfarbstoff gemäß Ansprüchen 1 oder 2, **dadurch gekennzeichnet, dass**
R¹ für Wasserstoff, Fluor, Chlor, COOH oder COOCH₃ steht,
R² für Wasserstoff, Fluor, Chlor, CF₃, oder CN steht,
R³ für Wasserstoff, Chlor, COOCH₃ oder CN steht,
R⁴ für Methyl oder Phenyl steht
und
R⁵ und R⁶ für Methyl
stehen.

4. Methinfarbstoff gemäß wenigstens einem der Ansprüche 1 bis 3, **dadurch gekennzeichnet, dass** er der Formel (I) gemäß Anspruch 1 entspricht, worin
R¹ für -COOCH₃, R² und R³ für Cl, R⁴ für -CH₃ und R⁵ und R⁶ für -CH₃ stehen, oder
R¹ für -COOCH₃, R² für H, R³ für Cl, R⁴ für -CH₃ und R⁵ und R⁶ für -CH₃ stehen, oder
R¹ für -COOCH₃, R² für Cl, R³ für H, R⁴ für -CH₃ und R⁵ und R⁶ für -CH₃ stehen, oder
R¹ für -COOCH₃, R² für H, R³ für -COOCH₃, R⁴ für -CH₃ und R⁵ und R⁶ für -CH₃ stehen, oder
R¹ für -COOCH₃, R² für H, R³ für CN, R⁴ für -CH₃ und R⁵ und R⁶ für -CH₃ stehen, oder
R¹ für -COOCH₃, R² für F, R³ für H, R⁴ für -CH₃ und R⁵ und R⁶ für -CH₃ stehen, oder
R¹ für -COOCH₃, R² für CF₃, R³ für H, R⁴ für -CH₃ und R⁵ und R⁶ für -CH₃ stehen, oder
R¹ für -Cl, R² und R³ für Cl, R⁴ für -CH₃ und R⁵ und R⁶ für -CH₃ stehen, oder
R¹ für -F , R² und R³ für Cl, R⁴ für -CH₃ und R⁵ und R⁶ für -CH₃ stehen.

5. Verwendung von mindestens einem Methinfarbstoff gemäß wenigstens einem der Ansprüche 1 bis 4 zum Massefärben von Kunststoffen.

6. Verwendung gemäß Anspruch 5, **dadurch gekennzeichnet, dass** es sich um mindestens einen Kunststoff aus der Reihe der Vinylpolymere, Polyester, Polyolefine, Polycarbonate und Polyamide handelt.

7. Verwendung gemäß wenigstens einem der Ansprüche 5 oder 6, **dadurch gekennzeichnet, dass** es sich bei dem Kunststoff um Polyamid 6 und/oder Polyamid 6.6 handelt.

8. Verwendung gemäß wenigstens einem der Ansprüche 5 bis 7, **dadurch gekennzeichnet, dass** der Methinfarbstoff in einer Menge von 0,0001 bis 1 Gew.-%, insbesondere 0,01 bis 0,5 Gew.-%, bezogen auf die Menge an Kunststoff eingesetzt wird.

9. Verfahren zum Massefärben von Kunststoffen, **dadurch gekennzeichnet, dass** mindestens ein Methinfarbstoff gemäß wenigstens einem der Ansprüche 1 bis 4 mit mindestens einem Kunststoff, vorzugsweise in Granulatform, trocken vermischt oder vermahlen wird und dieses Gemisch geschmolzen und homogenisiert wird.

10. Verfahren zum Massefärben von Kunststoffen, **dadurch gekennzeichnet, dass** mindestens ein Methinfarbstoff gemäß wenigstens einem der Ansprüche 1 bis 4 zu einer geschmolzenen, mindestens einen Kunststoff enthaltenden Kunststoffmasse zugegeben und diese dann homogenisiert wird.

11. Verfahren zum Massefärben von Kunststoffen, **dadurch gekennzeichnet, dass** mindestens ein Methinfarbstoff gemäß wenigstens einem der Ansprüche 1 bis 4 mit den monomeren Ausgangskomponenten zur Herstellung mindestens eines Kunststoffes vermischt wird und die Mischung anschließend polymerisiert wird.

12. Verfahren zum Massefärben von Polymethylmethacrylat (PMMA) **dadurch gekennzeichnet, dass** mindestens ein Methinfarbstoff gemäß wenigstens einem der Ansprüche 1 bis 4 mit mindestens einem Methacrylsäure-methylestermonomer vermischt oder darin gelöst wird und diese Mischung oder Lösung dann in Gegenwart mindestens eines Polymerisationskatalysators polymerisiert wird.

13. Kunststoffzusammensetzung, insbesondere Polyamidzusammensetzung, **dadurch gekennzeichnet, dass** sie mindestens einen Methinfarbstoff gemäß wenigstens einem der Anspüche 1 bis 4 enthalten.

14. Formteile, **dadurch gekennzeichnet, dass** sie mindestens eine Kunststoffzusammensetzung gemäß Anspruch 13 enthalten.

15. Verfahren zur Herstellung mindestens eines Methinfarbstoffs gemäß Ansprüchen 1 bis 4, **dadurch gekennzeichnet, dass** mindestens ein Aldehyd der Formel (II) 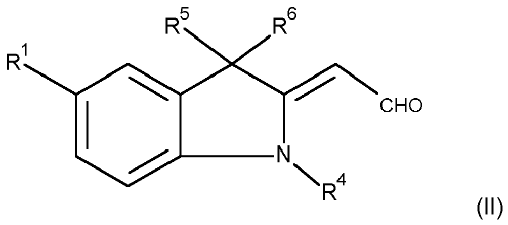 worin
R¹ für Wasserstoff, Halogen, COOH oder COOR⁷ steht,
R⁴ für Alkyl oder Phenyl steht
und
R⁵ und R⁶ unabhängig voneinander für Alkyl stehen,
und R⁷ für Alkyl steht,
mit mindestens einem Phenylacetonitril -Derivat der Formel (III) 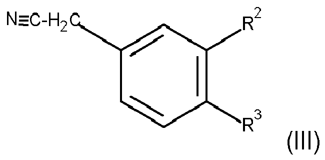 worin
R² für Wasserstoff, Halogen, CF₃, oder CN steht, R³ für Wasserstoff, Halogen, COOR⁸ oder CN steht, und R⁸ für Alkyl steht,
umgesetzt wird.

16. Aldehyd der Formel (II) 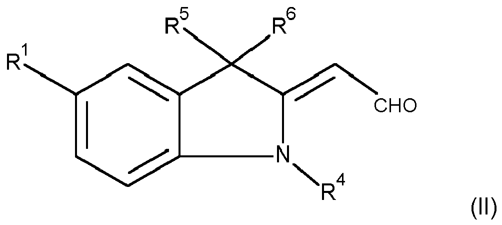 worin
R¹ für Wasserstoff, Halogen, COOH oder COOR⁷ steht,
R⁴ für Alkyl oder Phenyl steht
und
R⁵ und R⁶ unabhängig voneinander für Alkyl stehen,
und R⁷ für Alkyl steht.
